# EUROPEAN PATENT APPLICATION

(11) **EP 4 242 297 A1**
(43) Date of publication of application: **13.09.2023**
(21) Application number: 21888339.5
(22) Date of filing: 29.09.2021
(51) Int. Cl.: C12N 5/078, C12N 5/0789

(54) **CELL PREPARATION, USE OF PROTEIN IN CHARACTERIZING HEMATOPOIETIC STEM CELLS, AND METHOD FOR DETERMINING HEMATOPOIETIC STEM CELLS**

(30) Priority: 03.11.2020 CN 202011208703
(71) Applicant: EdiGene (GuangZhou) Inc., Guangzhou City, Guangdong 510000 (CN)
(72) Inventor: FANG, Riguo, Beijing 102206 (CN); MA, Kuiying, Beijing 102206 (CN); LI, Chao, Beijing 102206 (CN); YUAN, Pengfei, Beijing 102206 (CN)
(74) Representative: Lavoix
(86) International application number: PCT/CN2021/121702
(87) International publication number: WO 2022/095642

(57) **Abstract**

Provided are a cell preparation, a use of a protein in characterizing hematopoietic stem cells, and a method for determining hematopoietic stem cells. The cell preparation is CD34+, CD90+, CD45RA- sorted and obtained from among cells to be sorted, and hematopoietic stem cells that are positive for the following proteins, the protein being selected from one or more among CD48, CD66a, CD66c, CD66d, CD66e, and CD200. By using the foregoing protein(s) to characterize hematopoietic stem cells, not only can target cells be more accurately calibrated and evaluated in vitro, but the ability of a patient to maintain long-term reconstruction of the hematopoietic system after receiving a hematopoietic stem cell transplant can also be improved, achieving a better treatment effect.

## Description

### FIELD OF THEAPPLICATION

The present application relates to the technical field of biology, and in particular, to a cell preparation, use of a protein in identifying hematopoietic stem cells, and a method for identifying hematopoietic stem cells.

### BACKGROUND ART

Hematopoietic stem cells (HSCs) are a type of blood cells existing in bone marrow and blood with the ability of self-renewal and multi-lineage differentiation into all types of blood cells. In the hematopoietic system, hematopoietic stem cells are the source of differentiation of all cells, and have the potential of pluripotency and self-renewal. Hematopoietic stem cells include long-term (LT) HSCs and short-term (ST) HSCs, of which LT-HSCs are less in number and mostly exist in a resting state, and are mainly responsible for the long-term reconstruction capacity of the hematopoietic system. ST-HSCs has strong proliferative ability and can further differentiate into hematopoietic progenitor cells (HPCs). These progenitor cells retain some of the ability of lineage differentiation of the hematopoietic system, and can further differentiate into mature lymphocytes, myeloid cells, red blood cells, etc.

In the past four decades, with the progressive understanding of the hematopoietic system, hematopoietic stem cells have been increasingly used in clinical practice for cell therapy and gene therapy for blood disorders and immunodeficiency diseases. HSCs can be classified into cord blood (CB), mobilized peripheral blood (mPB) and bone marrow (BM) HSCs according to cell sources. Current indications for hematopoietic stem cell transplantation include:
(1) hematological malignancies, such as chronic myelocytic leukemia, acute myeloid leukemia, acute lymphoblastic leukemia, chronic myeloid leukemia, chronic lymphocytic leukemia, acute promyelocytic leukemia, non-Hodgkin's lymphoma, Hodgkin's lymphoma, myeloma, multiple myeloma, myelofibrosis and myelodysplastic syndrome, Burkitt's lymphoma, B-cell lymphoma, follicular lymphoma, mantle cell lymphoma, T-cell lymphoma, plasmablastic lymphoma, cutaneous T-cell lymphoma, primary macroglobulinemia, plasma cell leukemia, plasmablastic lymphoma, hairy cell leukemia, systemic mastocytosis, embryonic plasmacytoid dendritic cell tumor, and other hematologic malignancies;
(2) hematologic non-malignancies, such as aplastic anemia, fanconi anemia, thalassemia, sickle cell anemia, myelofibrosis, severe paroxysmal nocturnal hemoglobinuria, amegakaryocytic thrombocytopenia, and other hematologic non-malignancies;
(3) solid tumors, such as breast cancer, ovarian cancer, testicular cancer, kidney cancer, neuroblastoma, small cell lung cancer, germ cell tumors, Ewing's sarcoma, soft tissue sarcoma, Wilms' tumor, osteosarcoma, medulloblastoma, malignant brain tumors, and other solid tumors;
(4) diseases of the immune system, such as severe combined immunodeficiency, severe autoimmune diseases, primary central nervous system lymphoma, Wiskott-Aldrich syndrome, chronic granuloma, IPEX (immune dysregulation, polyendocrinopathy, enteropathy, X-linked) syndrome, AL amyloidosis, POEMS syndrome, hemophagocytic syndrome, rheumatoid arthritis, multiple sclerosis, systemic sclerosis, systemic lupus erythematosus, Crohn's disease, polymyositis and dermatomyositis, and other autoimmune, immune dysregulation and immunodeficiency diseases;
(5) other genetic or metabolic diseases, such as mucopolysaccharidosis, congenital dyskeratosis, lysosomal metabolic diseases, spherical cell-like leukoencephalopathy, metachromatic leukodystrophy, X-linked adrenoleukodystrophy, and other genetic or metabolic diseases.

Due to the limitations of ethics and research models, our knowledge of human hematopoietic stem cells is limited to studies with reference to the mechanism of mouse HSCs and studies on transplantation of human HSCs on immunodeficient mouse models. However, the developmental mechanism of mice is quite different from that of humans. Thus, the understanding of the phenotype and function of adult human hematopoietic stem cells is still limited, and there is no authoritative study to define the true immunophenotype of adult human hematopoietic stem cells.

CD34 is widely expressed in immature hematopoietic cells and is currently most widely used clinically to identify and sort human HSCs for transplantation. However, CD34 positive cells still have heterogeneity, and contain both HSCs and HPCs that can only differentiate into a specific lineage, such as megakaryocyte-erythroid progenitor cells (MEP), lymphoid-myeloid progenitor cells (LMP), multipotent progenitor cells (MPP), multipotent lymphoid progenitor cells (MLP), committed myeloid progenitor cells (CMP), granulocyte-monocyte progenitor cells (GMP), committed lymphoid progenitor cells (CLP), etc. These hematopoietic stem/progenitor cells have many similarities and differences in phenotype, sternness and differentiation ability. Among them, only long-term HSCs (LT-HSCs) have the abilities of long-term self-renewal and also remodeling of all hematologic lineages in the body. In order to maintain the long-term reconstruction of the hematopoietic system after transplantation and achieve better and safer therapeutic effects, we need to find an improved method to define human HSCs by surface marker proteins.

Several studies have been conducted to discover marker proteins that define human HSCs using different methods. For example, the most classic CD90 (Thy1) was first discovered in human fetal bone marrow and is used to this day. Although HSCs are thought to be CD90 positive, CD90 negative cells have been reported to still contain HSCs with the potential of self-renewal and multi-lineage differentiation.

CD38 is a glycoprotein expressed on the surface of many immune cells, such as T cells, B cells, NK cells, etc. As it was reported to be expressed on non-HSCs, CD34+ CD38- has been applied to the definition of HSC by several studies. However, we and several other project teams have found that the expression of CD38 is dramatically reduced after HSCs are cultured in vitro, so CD38 is not a surface marker protein that can well define HSCs.

Then, on the basis of CD34+ CD38- CD90+, a further study found that CD45RA could also be used to distinguish HSCs from HPCs. Lin-CD34+CD38-CD45RA-CD90+ enriched more human HSCs in both mPB and CB derived cells.

By screening different adhesion proteins on HSPCs, CD49f (integrin α6, ITGA6) was found to be a specific marker for HSCs. Lin-CD34+CD38-CD45RA- cells were further divided into CD90+CD49f+ and CD90+CD49f- for in vivo function validation, CD90+CD49f+ showed more than 6-fold higher transplantation efficiency than CD90+CD49- cells, and only CD90+CD49f+ could show a long-lasting hematopoietic reconstitution effect during continuous transplantation.

Another report found that CD166 is a functional marker protein on both mouse and human HSCs. Human CB CD34+ HSPCs are mostly CD166 positive. In addition, osteoblasts (OBs), which are very important to affect HSCs in the hematopoietic microenvironment, also express CD166. In Lin-CD34+CD38- cells, the CD166+ sub-population showed higher transplantation efficiency than the CD166- sub-population, and this transplantation advantage may be achieved through the homotypic interaction of CD166 on HSCs and OBs.

CD105 (endoglin) is widely recognized as a marker protein for endothelial cells, but it is also highly expressed in 30-80% of human CD34+ HSPCs. Both CD105 and CD34 positively regulate TGF-betal on hematopoietic cells to inhibit the cell cycle and maintain the stem/progenitor cells in a more primitive state. CD34+ CD105+ marks early developmental, more sternness human hematopoietic stem cells.

In another study, CD201 (endothelial protein C receptor, EPCR) was used in combination with CD34 and CD45RA to define cultured human HSCs. The EPCR/PAR1 pathway has been reported to promote LT-HSCs retention in bone marrow by limiting nitric oxide production and regulating Cdc42 activity. Also, EPCR was used to define mouse HSCs in mouse bone marrow.

In addition, this group further found that there were still two functional sub-populations of CD34+CD45RA-EPCR+ cells in cultured CB, with LT-HSCs mainly concentrated in the Integrin-a3 (ITGA)+ sub-population. ITGA3+ cells showed a stronger multi-lineage differentiation potential, continuous reconstruction in an immunodeficient mouse model and HSC-specific gene expression. Thus, EPCR+CD90+CD133+CD34+CD45RA-ITGA3+ cells are considered to be closer to the real definition of HSCs.

In another study, glycoprotein (endomucin, EMCN) was identified as another potential marker molecule for human HSCs by gene expression analysis of human bone marrow samples. It is highly expressed in human HSCs but its expression level decreases during the process of differentiation into progenitor cells. Previously, EMCN was also found to be specifically expressed in mouse HSCs during embryonic development, but not in erythroid progenitors, further demonstrating that EMCN may serve as a potential surface marker for HSCs.

With the development of single-cell sequencing, several research groups have further grouped the stem cells and progenitor cells of human HSPCs at various stages and analyzed their gene lineage. CD164 was found to be expressed in the earliest lineage of the hematopoietic system, and was able to clearly distinguish between early and late stem/progenitor cells. Compared with CD34+CD164low sub-population, CD34+CD164hi sub-population has stronger colony-forming ability, in vitro differentiation ability, expansion ability and in vivo whole lineage differentiation ability. Also, CD164 has more identifiability than CD90 + or CD38-, which traditionally defined LT-HSCs.

In addition, it has been reported that some surface proteins such as HLA-DR, c-Kit, Tie2 and IL-3R are also highly expressed on HSPCs, but could not be used to define a sub-population with a better sternness in Lin-CD34+CD38-CD45RA-CD90+ cells.

In the above studies, marker proteins of human HSCs have been found by different methods. However, the real definition of immunophenotype of HSCs is still controversial, which greatly affects the clinical development of hematopoietic stem cell transplantation and the evaluation of its therapeutic efficacy and safety.

### SUMMARY OF THE APPLICATION

To overcome the above problems, the present application provides a method for identifying hematopoietic stem cells by proteins, the proteins include CD34, CD90, and CD45RA; and
one or two or more protein(s) selected from the group consisting of CD48, CD66a, CD66c, CD66d, CD66e and CD200. After characterizing hematopoietic stem cells using proteins, not only can the target cells be more accurately labeled and evaluated in vitro, but also can focus on improving the patient's ability to maintain long-term reconstruction of the hematopoietic system after receiving hematopoietic stem cell transplantation, thereby achieving better therapeutic effect.

The significance of using the above proteins to define hematopoietic stem cells is as follows:
(1) Cell purity and quality of HSCs from various sources can be more effectively evaluated and controlled in vitro.
(2) As the number of hematopoietic stem cells that can be obtained in the human body is limited and often does not meet the minimum number criteria for clinical transplantation, in vitro expansion culture is thus required. However, most culture systems cannot well maintain the stemness, resulting in a decreased proportion of HSCs after culture. By labeling HSCs more accurately with marker proteins, it is also possible to better evaluate and optimize the in vitro culture system.
(3) Based on the ability to reconstruct the whole hematopoietic system, HSCs are used for gene editing to treat a variety of blood and immune disorders. In the process of genetic modification, labeling HSCs more accurately by marker proteins can identify the target cells more accurately, achieving targeted HSC genetic modification.
(4) HSC marker proteins are not only crucial for understanding the physiological development of the hematopoietic system, but also make it difficult to target and eradicate due to many refractory malignant hematological tumors that undergo cancerous transformation on HSCs. Therefore, more accurate labeling of HSCs by marker proteins will help to better understand the pathogenesis of these tumors and expand therapeutic targets.
(5) For HSC marker proteins, corresponding detection products such as antibodies, detection magnetic beads and detection kits can be developed to enrich HSCs and provide more effective HSCs for scientific research and clinical application.

Thus, the present application provides the following technical solutions:
1. Use of a protein in identifying hematopoietic stem cells, wherein the protein comprises
   (1) CD34, CD90 and CD45RA; and
   (2) one, two, three, four or more protein(s) selected from the group consisting of CD48, CD66a, CD66c, CD66d, CD66e and CD200.
2. The use of item 1, wherein the protein comprises
   (1) CD34, CD90 and CD45RA; and
   (2) one, two or three protein(s) selected from CD66a, CD66e and CD200, preferably CD66e.
3. A method for identifying hematopoietic stem cells, comprising the following steps:
   detecting whether the cells to be tested are CD34 positive;
   detecting whether the cells to be tested are CD90 positive;
   detecting whether the cells to be tested are CD45RA negative; and
   detecting whether the cells to be tested are positive for one, two, three, four or more protein(s) selected from the group consisting of CD48, CD66a, CD66c, CD66d, CD66e and CD200, and
   identifying the cells to be tested as hematopoietic stem cells if the cells to be tested are CD34 positive, CD90 positive, CD45RA negative, and positive for one, two, three, four or more protein(s) selected from the group consisting of CD48, CD66a, CD66c, CD66d, CD66e and CD200.

The above-mentioned detection steps are intended to describe the items to be detected, and are not intended to limit the order of detection implementation, that is to say, the embodiments of the present application cover all the technical solutions for the above-mentioned detection steps to be carried out in sequential order, not in sequential order, and simultaneously.
4. The method of item 3, wherein the cells to be tested are derived from cord blood, recruited peripheral blood, or bone marrow.
5. The method of item 3 or 4, wherein the protein is one, two, or three proteins selected from a group consisting of CD66a, CD66e, and CD200, preferably CD66e.
6. A method for screening hematopoietic stem cells, comprising:
   screening hematopoietic stem cells of CD34+, CD90+, CD45RA- and positive for one, two, three, four or more protein(s) from cells to be isolated;
   wherein the protein(s) is one, two, three, four or more protein(s) selected from the group consisting of CD48, CD66a, CD66c, CD66d, CD66e and CD200.
7. The method of item 6, wherein the cells to be isolated are derived from cord blood, recruited peripheral blood, or bone marrow.
8. The method of item 6 or 7, wherein the protein is one, two, or three proteins selected from a group consisting of CD66a, CD66e, and CD200, preferably CD66e.
9. A cell preparation, comprising hematopoietic stem cells sorted from cells and having CD34+, CD90+, CD45RA-, and being positive for one, two, three, four or more protein(s) selected from the group consisting of CD48, CD66a, CD66c, CD66d, CD66e and CD200.
10. The cell preparation of item 9, wherein the cells are derived from cord blood, recruited peripheral blood, or bone marrow.
11. The cell preparation of item 9 or 10, wherein the protein is one, two or three protein(s) selected from CD66a, CD66e, and CD200, preferably CD66e.
12. A method for preparing a cell preparation, comprising:
   screening hematopoietic stem cells of CD34+, CD90+, CD45RA- and positive for one, two, three, four or more protein(s) from cells to be sorted;
   wherein the protein(s) is one, two, three, four or more selected from the group consisting of CD48, CD66a, CD66c, CD66d, CD66e and CD200.
13. The method of item 12, wherein the cells to be sorted are derived from cord blood, recruited peripheral blood, or bone marrow.
14. The method of item 12 or 13, wherein the protein is one, two or three protein(s) selected from CD66a, CD66e, and CD200, preferably CD66e.
15. A medicament for improving hematopoietic reconstruction, comprising the cell preparation of any one of items 9 to 11 or a cell preparation prepared by the method of any one of items 12 to 14.
16. A method for reconstructing the hematopoietic system of a subject, comprising administering to the subject the cell preparation of any one of items 9 to 11 or a cell preparation prepared by the method of any one of items 12 to 14.

In one embodiment, the present application further relates to use of the cell preparation of any one of items 9-11 or a cell preparation prepared by the method of any one of items 12-14 in preparation of a medicament for reconstructing the hematopoietic system of a subject.
17. A kit or product for detecting hematopoietic stem cells, comprising a compound for:
   detecting whether the cells to be tested are CD34 positive;
   detecting whether the cells to be tested are CD90 positive;
   detecting whether the cells to be tested are CD45RA negative; and
   detecting whether the cells to be tested are positive for one, two, three, four or more protein(s) selected from the group consisting of CD48, CD66a, CD66c, CD66d, CD66e and CD200.
18. The kit or product of item 17, wherein the protein is one, two or three protein(s) selected from CD66a, CD66e and CD200, preferably CD66e.
19. The kit or product of item 17 or 18, wherein the cells to be tested are derived from cord blood, recruited peripheral blood or bone marrow.
20. The kit or product of any one of items 17-19, wherein the compound is an antibody.
21. The kit or product of item 20, wherein the antibody is a chemically labelled antibody, such as a luminescent compound labelled antibody, such as a fluorescent labelled antibody.
22. The kit or product of item 20, wherein the antibody is an antibody attached to a magnetic bead.
23. A kit or product for sorting or enriching hematopoietic stem cells, comprising a compound for sorting hematopoietic stem cells from cells, wherein the hematopoietic stem cells are CD34+, CD90+, CD45RA-, and positive for one, two, three, four or more protein(s) selected from the group consisting of CD48, CD66a, CD66c, CD66d, CD66e and CD200.
24. The kit or product of item 23, wherein the protein is one, two or three protein(s) selected from CD66a, CD66e and CD200, preferably CD66e.
25. The kit or product of item 23 or 24, wherein the cells are derived from cord blood, recruited peripheral blood or bone marrow.
26. The kit or product of any one of items 23-25, wherein the compound is an antibody.
27. The kit or product of item 26, wherein the antibody is a chemically labelled antibody, such as a luminescent compound labelled antibody, such as a fluorescent labelled antibody.
28. The kit or product of item 26, wherein the antibody is an antibody attached to a magnetic bead.
29. A method for screening a culture medium for hematopoietic stem cells, comprising culturing hematopoietic stem cells in a culture medium to be screened, and detecting maintenance or elevation of proportion of hematopoietic stem cells characterized by CD34+, CD90+, CD45RA-, and positive for one, two, three, four or more protein(s) selected from the group consisting of CD48, CD66a, CD66c, CD66d, CD66e and CD200, wherein the maintenance or elevation of proportion of hematopoietic stem cells characterized by CD34+, CD90+, CD45RA-, and positive for one, two, three, four or more protein(s) selected from the group consisting of CD48, CD66a, CD66c, CD66d, CD66e and CD200 after cultivation indicates that the culture medium to be screened is a suitable culture medium for hematopoietic stem cells.
30. The method of item 29, wherein the protein is one, two, or three proteins selected from a group consisting of CD66a, CD66e, and CD200, preferably CD66e.
31. A culture medium for hematopoietic stem cells screened by the method of item 29 or 30.
32. A method for treating a disease in a subject, comprising administering to the subject an effective amount of the cell preparation of any one of items 9 to 11 or a cell preparation prepared by the method of any one of items 12 to 14.
33. The method of item 32, wherein the disease is a disease requiring hematopoietic stem cell therapy to reconstruct the hematopoietic system of the subject.
34. The method of item 33, wherein the disease is a malignancy, a hematological disease, or an immune system disease.
35. The method of item 34, wherein the disease comprises:
   (1) chronic myelocytic leukemia, acute myeloid leukemia, acute lymphoblastic leukemia, chronic myeloid leukemia, chronic lymphocytic leukemia, acute promyelocytic leukemia, non-Hodgkin's lymphoma, Hodgkin' s lymphoma, myeloma, multiple myeloma, myelofibrosis and myelodysplastic syndrome, Burkitt's lymphoma, B-cell lymphoma, follicular lymphoma, mantle cell lymphoma, T-cell lymphoma, plasmablastic lymphoma, cutaneous T-cell lymphoma, primary macroglobulinemia, plasma cell leukemia, plasmablastic lymphoma, hairy cell leukemia, systemic mastocytosis, embryonic plasmacytoid dendritic cell tumor, and other hematologic malignancies;
   (2) aplastic anemia, fanconi anemia, thalassemia, sickle cell anemia, myelofibrosis, severe paroxysmal nocturnal hemoglobinuria, amegakaryocytic thrombocytopenia, and other hematologic non-malignancies;
   (3) breast cancer, ovarian cancer, testicular cancer, kidney cancer, neuroblastoma, small cell lung cancer, germ cell tumors, Ewing's sarcoma, soft tissue sarcoma, Wilms' tumor, osteosarcoma, medulloblastoma, malignant brain tumors, and other solid tumors;
   (4) severe combined immunodeficiency, severe autoimmune diseases, primary central nervous system lymphoma, Wiskott-Aldrich syndrome, chronic granuloma, IPEX (immune dysregulation, polyendocrinopathy, enteropathy, X-linked) syndrome, AL amyloidosis, POEMS syndrome, hemophagocytic syndrome, rheumatoid arthritis, multiple sclerosis, systemic sclerosis, systemic lupus erythematosus, Crohn's disease, polymyositis and dermatomyositis, and other autoimmune, immune dysregulation and immunodeficiency diseases; or
   (5) mucopolysaccharidosis, congenital dyskeratosis, lysosomal metabolic diseases, spherical cell-like leukoencephalopathy, metachromatic leukodystrophy, X-linked adrenoleukodystrophy, and other genetic or metabolic diseases.
36. The present application further relates to use of the cell preparation of any one of items 9-11 or a cell preparation prepared by the method of any one of items 12-14 in preparation of a medicament for treating the above-mentioned diseases.

### EFFECT OF THE APPLICATION

The protein provided by the present application characterizes hematopoietic stem cells, which not only enables the target cells to be more accurately labeled and evaluated in vitro, but also can focus on improving the patient's ability to maintain long-term reconstitution of the hematopoietic system after receiving hematopoietic stem cell transplantation, thereby achieving better therapeutic effects.

### BRIEF DESCRIPTION OF THE DRAWINGS

Fig. 1 is a flow chart of surface protein expression verification in Example 3.
Fig. 2 is a schematic diagram of the difference in expression levels of different surface proteins on LT-HSCs and HSPCs using flow cytometry in Example 3.
Figs. 3A to 3C are schematic diagrams of detection of the expression of CD66 (a, c, d, e), CD48 and CD200 on human HSPCs and LT-HSCs using flow cytometry in Example 3.
Figs. 4A-4B are schematic diagrams of the differences in LT-HSCs contained in HSPCs cultured in vitro or uncultured in Example 4.
Figs. 5A-5B are schematic diagrams of the rescreening of surface marker proteins on HSPCs from different sources in Example 4.
Figs. 6A-6B are schematic diagrams of using the target protein to distinguish different sub-populations of HSPCs and analyzing the proportions of LT-HSCs in the sub-populations in Example 4.
Figs. 7A-7F are schematic diagrams comparing the proportions of LT-HSCs contained in CD200, CD66a, and CD66e sub-populations over culture time in Example 4.
Figs. 8A-8B are schematic diagrams of flow sorting of CD34+CD90+CD45RA-CD66e- or CD34+CD90+CD45RA-CD66e hi cells (CD66e hi refers to high expression of CD66e) and flow cytometric analysis of cell purity after sorting in Example 5, where 8A is a schematic diagram of flow sorting of CD34+CD90+CD45RA-CD66e- or CD34+CD90+CD45RA-CD66e hi cells, and 8B is a schematic diagram of cell purity detection after flow sorting.
Fig. 9 is a schematic diagram of HSC-related gene expression levels of CD34+, CD34+CD90+CD45RA-CD66e-, and CD34+CD90+CD45RA-CD66e hi cells detected by qRT-PCR in Example 5, respectively.
Fig. 10 is a statistical diagram of cell transplantation efficiency in peripheral blood of mice at 4, 12 and 16 weeks after cell transplantation in Example 6.
Fig. 11 is a schematic diagram of HSCs with reconstitution ability in two groups of cells calculated using finite dilution method in Example 6.
Fig. 12 is a schematic diagram of the transplantation situation in the bone marrow and spleen at the 16th weeks after transplantation in Example 6.
Fig. 13 is a schematic diagram of the transplantation situation in the peripheral blood, bone marrow and spleen of mice in Example 7.

### DETAILED DESCRIPTION OF THE APPLICATION

The present application is described below in detail in conjunction with the embodiments illustrated in the accompanying drawings, wherein the same numbers in all the attached drawings represent the same characteristics. Although specific embodiments of the present application are shown in the accompanying drawings, it should be understood that the application can be implemented in various forms and should not be limited by the embodiments described herein. On the contrary, these embodiments are provided in order to have a more thorough understanding of the present application and to fully convey the scope of the present application to those skilled in the art.

It is noted that certain terms are used throughout the specification and claims to refer to particular components. Those skilled in the art should understand that a skilled artisan may use different terms to refer to the same component. The description and claims do not take differences in terms as a way for distinguishing components, but rather take differences in functionality as a criterion for distinguishing components. As used throughout the description and claims, the terms "comprises/comprising" and "includes/including" are open-ended terms, thus they should be interpreted as "including, but not limited to". The following description is of preferred embodiments for practicing the application, however, the description is for the purpose of illustrating the general principles of the application and is not intended to limit the scope of the application. The scope of protection of the present application should be determined by the attached claims.

Use of a protein(s) in identifying hematopoietic stem cells is provided, wherein the protein(s) comprises one or two or more selected from the group consisting of CD48, CD66a, CD66c, CD66d, CD66e and CD200.

In some embodiments, the present application relates to use of proteins in identifying hematopoietic stem cells, wherein the proteins comprise (1) CD34, CD90, and CD45RA; and
(2) one, two, three, four or more protein(s) selected from the group consisting of CD48, CD66a, CD66c, CD66d, CD66e and CD200.

CD48 is a CD2 subfamily protein of the immunoglobulin family. It is not a transmembrane protein but can be anchored to the cell membrane by GPI, and its C-terminal domain can be cleaved to form a soluble protein. CD48 is expressed on a variety of immune cells and endothelial cells etc., and binds with high affinity to the 2B4 receptor on NK cells and with low affinity to CD2. CD48 may promote the interaction between activated lymphocytes and participate in T cell activation.

The CEA family contains two subfamilies, the CEA cell adhesion molecule and the pregnancy-specific polysaccharide, and is located on the long arm of chromosome 19. The characteristic of CEACAM proteins are that they can anchor on the cell membrane, and they contain a 34-amino-acid signal peptide in the Ig domain. They contain 12 member proteins (CEACAM1, CEACAM3, CEACAM4, CEACAM5, CEACAM6, CEACAM7, CEACAM8, CEACAM16, CEACAM18, CEACAM19, CEACAM20, CEACAM21).

CD66a (CEACAM1), the most widely distributed member of the CEACAM family, contains both secretory and transmembrane forms, and its function mainly depends on the N-terminal domain. CD66a was originally discovered in the hepatic bile duct as a bile glycoprotein. Later studies have reported that it is also expressed in lymphocytes, epithelial cells, endothelial cells, etc. As an intercellular adhesin, it regulates cell functions through homophilic or heterophilic connections and participates in cell differentiation, tissue structure formation, angiogenesis, apoptosis, tumor suppression, metabolism, innate immunity and adaptive immunity.

CD66c (CEACAM6) belongs to the CEA family, and binds to the cell membrane via glycosyl phosphatidyl inositol (GPI). Physiologically, CD66c is significantly overexpressed in a range of epithelial cells, neutrophils and monocytes. At the same time, CD66c is also commonly used for serum detection of malignancies.

CD66d (CEACAM3) belongs to the CEA family, and is located on human chromosome 19q13.2. Various bacterial pathogens bind to and invade host cells through this protein. Therefore, CD66d plays an important role in the regulation of innate immunity against pathogen infection.

CD66e (CEACAM5) belongs to the CEA family, and binds to the cell membrane via glycosyl phosphatidyl inositol (GPI). Expression of CD66e begins in the early stages of human embryonic and fetal development (9-14 weeks) and is maintained throughout life in specific cells. In adult tissues, CD66e is expressed in the intestine, stomach, tongue, esophagus, cervix, sweat glands and prostate. Cd66e is also a commonly used tumor recognition marker, but its mechanism and function are unclear.

CD200 molecule is an I-type membrane glycoprotein with a molecular weight of (41-47) kDa and a typical V/C2 structure. It is composed of an extracellular signal peptide, a dihydroxy dehydrogenase region, a V region, a C2 region, a transmembrane region and an intracellular region, and belongs to the immunoglobulin superfamily (IgSF). In human, it is located on chromosome 3q12-q13 and consists of 6 exons and 5 introns. After transcription, the full length of the mRNA is 2.14kb, encoding a polypeptide chain of 269 amino acids. In mouse, CD200 is located on chromosome 16 region B5, with a full length of 2.20 kb, encoding a polypeptide chain of 278 amino acids.

In a preferred embodiment of the application, the protein(s) is CD66a, CD66e and/or CD200, preferably CD66e.

The present application provides a method for identifying hematopoietic stem cells, comprising the following steps:
detecting whether the cells to be tested are CD34 positive;
detecting whether the cells to be tested are CD90 positive;
detecting whether the cells to be tested are CD45RA negative; and
detecting whether the cells to be tested are positive for one or two or more protein(s) selected from the group consisting of CD48, CD66a, CD66c, CD66d, CD66e and CD200, and
identifying the test cells as hematopoietic stem cells if the tested cells are CD34 positive, CD90 positive, CD45RA negative and positive for the proteins described above.

The above testing steps are intended to describe the items that need to be tested and are not intended to limit the order in which the tests are performed.

The present application does not limit the source of cells to be tested, and the cells to be tested may, for example, be derived from cord blood, recruited peripheral blood or bone marrow.

The cells to be tested refer to cells obtained by sorting hematopoietic stem cells derived from cord blood, recruited peripheral blood or bone marrow.

The present application does not impose any limitations on its operating methods, and the sorting may be performed according to methods well-known to those skilled in the art, such as flow cytometry sorting, magnetic bead sorting, etc., depending on specific antibody staining.

In a preferred embodiment of the application, the protein(s) is CD66a, CD66e and/or CD200, preferably CD66e.

The present application characterizes hematopoietic stem cells by using the aforementioned proteins, not only can the target cells be more accurately labeled and evaluated in vitro, but also can focus on improving the patient's ability to maintain long-term reconstruction of the hematopoietic system after receiving hematopoietic stem cell transplantation, thereby achieving better therapeutic effect.

The present application provides a method for screening hematopoietic stem cells, comprising the following steps:
screening hematopoietic stem cells, which are CD34+, CD90+, CD45RA- and positive for protein from cells to be isolated;
wherein the protein is one or two or more protein(s) selected from the group consisting of CD48, CD66a, CD66c, CD66d, CD66e and CD200.

The hematopoietic stem cells refer to hematopoietic stem cells that are CD34 positive, CD90 positive, CD45RA negative and positive for one or two or more protein(s) selected from the group consisting of CD48, CD66a, CD66c, CD66d, CD66e and CD200.

The present application does not impose any limitations on the above-mentioned screening methods, and the screening may be performed according to methods well-known to those skilled in the art as needed, such as, flow cytometry, magnetic bead sorting, etc.

In a preferred embodiment of the present application, the cells to be isolated are derived from cord blood, recruited peripheral blood or bone marrow.

In a preferred embodiment of the present application, the protein(s) is CD66a, CD66e and/or CD200, preferably CD66e.

The present application provides a cell preparation comprising hematopoietic stem cells, which are CD34+, CD90+, CD45RA- and positive for protein(s), obtained by sorting from cells to be isolated, the protein(s) is one or two or more selected from the group consisting of CD48, CD66a, CD66c, CD66d, CD66e and CD200.

In a preferred embodiment of the present application, wherein the cells to be isolated are derived from cord blood, recruited peripheral blood or bone marrow.

In a preferred embodiment of the present application, the protein(s) is CD66a, CD66e and/or CD200, preferably CD66e.

The present application provides a method of preparing a cell preparation, comprising the following steps:
screening a cell preparation, which is CD34+, CD90+, CD45RA- and positive for protein(s), from cells to be sorted;
wherein the protein(s) is one or two or more selected from the group consisting of CD48, CD66a, CD66c, CD66d, CD66e and CD200.

The cell preparation refers to hematopoietic stem cells that are CD34 positive, CD90 positive, CD45RA negative and positive for one or two or more protein(s) selected from the group consisting of CD48, CD66a, CD66c, CD66d, CD66e and CD200.

In a preferred embodiment of the application, wherein the cells to be sorted are derived from cord blood, recruited peripheral blood or bone marrow.

In a preferred embodiment of the application, the protein(s) is CD66a, CD66e and/or CD200, preferably CD66e.

The present application provides a medicament for improving hematopoietic reconstruction, the medicament comprising the cell preparation as described above or a cell preparation prepared by the method as described above.

The present application provides a method of sorting hematopoietic stem cells, comprising the following steps:
sorting hematopoietic stem cells, which are CD34+, CD90+, CD45RA- and positive for protein, from cells to be isolated using a CD34 antibody, a CD90 antibody, a CD45RA antibody and a protein antibody;
wherein the protein is one or two or more protein(s) selected from the group consisting of CD48, CD66a, CD66c, CD66d, CD66e and CD200.

In a preferred embodiment of the application, the cells to be isolated are derived from cord blood, recruited peripheral blood or bone marrow.

In a preferred embodiment of the application, the protein(s) is CD66a, CD66e and/or CD200, preferably CD66e.

The present application also relates to a method of reconstructing the hematopoietic system of a subject, comprising administering to the subject the cell preparation as described above or a cell preparation prepared by the method as described above.

The present application also relates to a kit or product for detecting hematopoietic stem cells, comprising compounds for detecting:
detecting whether the cells to be tested are CD34 positive;
detecting whether the cells to be tested are CD90 positive;
detecting whether the cells to be tested are CD45RA negative; and
detecting whether the cells to be tested are positive for one, two, three, four or more protein(s) selected from the group consisting of CD48, CD66a, CD66c, CD66d, CD66e and CD200.

In some embodiments, the protein is one, two or three protein(s) selected from CD66a, CD66e and CD200, preferably CD66e. For the cells to be isolated, there are no specific restrictions in this application, they can be derived from cord blood, recruited peripheral blood or bone marrow or cultured cells. The present application does not impose any limitations on the above-mentioned screening methods, and the detection may be performed according to methods known to those skilled in the art, for example, detection depending on a specific antibody, flow cytometry detection, etc.

The present application further relates to a kit or product for sorting or enriching hematopoietic stem cells, comprising a compound for sorting hematopoietic stem cells from cells, wherein the hematopoietic stem cells are CD34+, CD90+, CD45RA- and positive for one, two, three, four or more protein(s) selected from the group consisting of CD48, CD66a, CD66c, CD66d, CD66e and CD200.

For the cells to be sorted or enriched, there are no specific restrictions in this application, and they can be derived from cord blood, recruited peripheral blood or bone marrow or cultured cells. The present application does not impose any limitations on the above-mentioned screening methods, and the detection may be performed according to methods known to those skilled in the art, for example, sorting depending on a specific antibody, such as flow cytometry detection, magnetic bead sorting, etc.

The present application further relates to a method for screening a culture medium for hematopoietic stem cells, comprising culturing hematopoietic stem cells in a culture medium to be screened, and detecting maintenance or elevation of proportion of hematopoietic stem cells characterized by CD34+, CD90+, CD45RA-, and positive for one, two, three, four or more protein(s) selected from the group consisting of CD48, CD66a, CD66c, CD66d, CD66e and CD200, wherein the maintenance or elevation of proportion of hematopoietic stem cells characterized by CD34+, CD90+, CD45RA-, and positive for one, two, three, four or more protein(s) selected from a group consisting of CD48, CD66a, CD66c, CD66d, CD66e and CD200 after cultivation indicates that the culture medium to be screened is a suitable culture medium for hematopoietic stem cells. The present application further relates to a culture medium for hematopoietic stem cells, the culture medium being screened by the method as described above.

The application further relates to a method of treating a disease in a subject, comprising administering to the subject an effective amount of the cell preparation as described above or a cell preparation prepared by the method as described above. In some embodiments, the disease is a disease requiring administration of hematopoietic stem cells to reconstitute the hematopoietic system of the subject.

### EXAMPLES

The present application provides a general and/or specific description of the materials and test methods used in the experiments. In the following examples, % means wt%, i.e., weight percentage, unless otherwise specified. The reagents or instruments used without specifying the manufacturer, are conventional reagent products commercially available.

### Example 1: Isolation of hematopoietic stem cells

The human hematopoietic stem cells used were mainly derived from cord blood. The cord blood sample, after being taken from the hospital, was rewarmed at room temperature for 0.5-1 h, and transferred from the blood collection bag to a 50 ml centrifuge tube, and gently mixed well. 100 µl sample was taken with pipette respectively for white blood cell counting and detection of CD34 positive proportion using flow cytometry. The remaining blood sample was diluted 1: 1 with 1% HAS in saline and gently mixed with pipette. The mixed solution was placed in a new 50 ml centrifuge tube, and an appropriate amount of lymphocyte separation solution was added to the bottom of the tube, and then centrifugation was performed at 400 g, accelerate 3 decelerate 0 for 30 min. After centrifugation, the centrifuge tube was lowly taken out, keeping the liquid level. The buffy coat cells were carefully pipetted into a new centrifuge tube, the cells were washed with 1% HAS in saline, and centrifuged at 500 g, accelerate 9 decelerate 5 for 10 minutes.

Cells were resuspended after centrifugation and sorted for CD34 positive cells (CD34+ cell magnetic bead sorting kit, Miltenyi, 130-046-703). First, 100 µl of FcR blocking antibody and 100 µ of CD34-beads per 1 × 10⁶ total cells were incubated at 4°C for 30 minutes. After incubation, the cells were washed with 40 ml of 1% HAS in saline, centrifuged at 400g for 8 min, and resuspended at 1 × 10⁹ cells per 3 ml.

The CD34+ cell sorting column was placed on a MACS magnetic separation rack, a 40 µm cell sieve was placed on the sorting column, and a 15 ml centrifuge tube was placed below the sorting column. The filter sieve was rinsed with 3 ml of 1% HAS in saline, and then added the cell suspension, after dripping, rinsed 3 times with 3 ml of 1% HAS in saline. The magnetic field was removed from the adsorption column, and 1% HAS in normal saline was added to collect the cells into the centrifuge tube. The operation was repeated once. The cells were mixed and counted, cultured or frozen. A portion of the cells was collected for flow cytometry to detect CD34 expression and confirm its purity.

### Specific information on each component of hematopoietic stem cell complete culture medium

### Example 2: Culture of hematopoietic stem cells

First, a complete culture medium for hematopoietic stem cells was prepared, and the components and their amounts are as follows.

**Table 1: Specific information on each component of hematopoietic stem cell complete culture medium**

| Components | Brand | Item NO. | Use concentration |
|---|---|---|---|
| StemSpan SFMEII | Stem cell | 09655 | |
| IL6 | PeproTech | 200-06 | 20 ng/ml |
| TPO | PeproTech | 300-18-100 | 100 ng/ml |
| FLT3L | PeproTech | AF-300-19-100 | 100 ng/ml |
| SCF | PeproTech | 300-07-100 | 100 ng/ml |
| Penicillin-streptomycin | Gibco | 15140122 | 1: 100 (P: 100 units/ml, S: 100 |
| | | | µg/ml) |

According to the counting result of Example 1, 5×10⁴ CD34+ cells were added to each well of a 24-well plate, and the cells were cultured in 1 ml of the complete culture medium. After three days of culture, cells were collected for flow cytometry.

### Example 3: Primary screening for surface protein expression

A human surface protein assay plate (BD Lyoplate-Human cell surface marker screening panel, BD Biosciences, CAT# 560747) was used for this assay. The assay plate was coated with 242 human surface marker proteins and 9 isotype control antibodies in each well of the 96-well plate.

A flow cytometry staining solution was prepared: PBS + 0.5% BSA + 5 mM EDTA. Fresh/cultured human HSCs were resuspended with the flow cytometry staining solution and added into the assay plate at 50000 cells/50 µl per well. After staining at 4°C for 30 minutes, 100 µl of the flow cytometry staining solution was added to each well, and the supernatant was centrifuged at 400 g. According to the type of the primary antibody in the plate as mouse antibody or rat antibody, Alexa Fluor^{®} 647-conjugated goat anti-mouse Ig or goat anti-rat Ig (both diluted by 1: 200 for use concentration) was used to stain at 4°C in dark for 30 min, and 100 µl of the flow cytometry staining solution was added to each well, followed by washing and centrifugation twice.

Then, Brilliant Violet 510 anti-human CD34 antibody (Biolegend, Code: 343528) and FITC anti-human CD90 (Thy1) antibody (Biolegend, Code: 328108) each was used to stain at a use concentration of 5 µl per 100 µl of cells for 15 minutes at 4°Cin dark. 100 µl of the flow cytometry staining solution was added to each well, centrifuged at 400 g to remove the supernatant. The cells were resuspended in 100 ul of the flow cytometry staining solution, and detected on a high throughput flow cytometer (BD LSRFortessa Flow Cytometry).

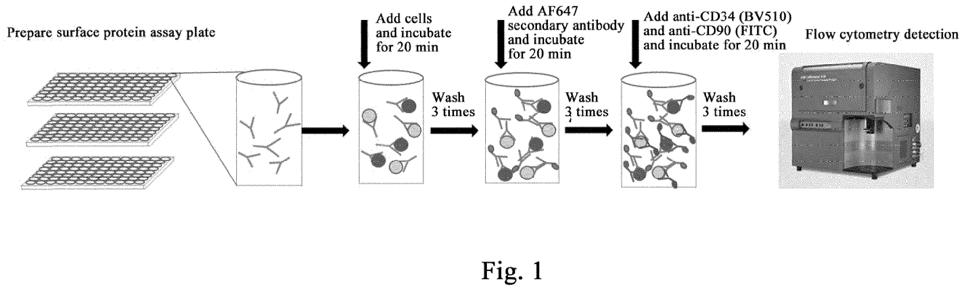

The process is shown in Fig. 1.

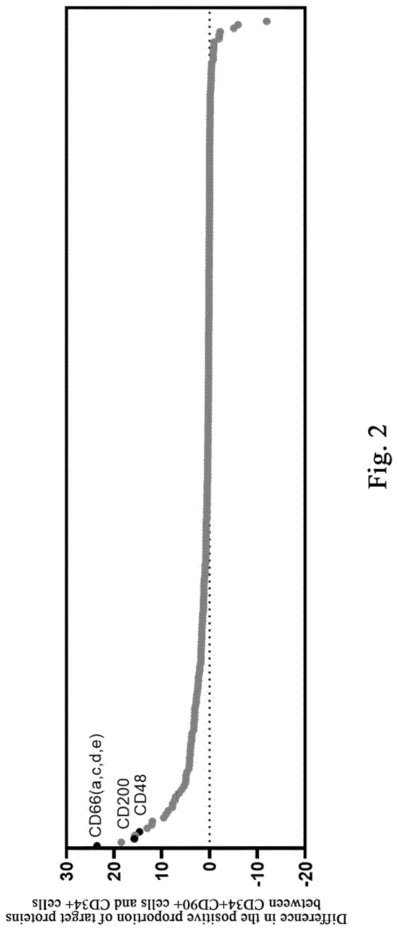

The results of preliminary screening for surface protein expression are shown in Fig. 2.

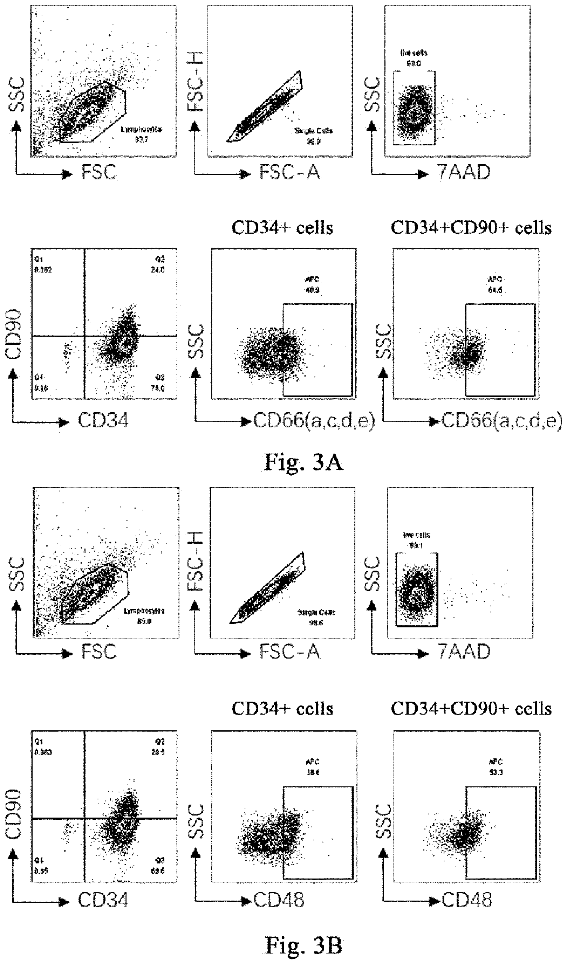

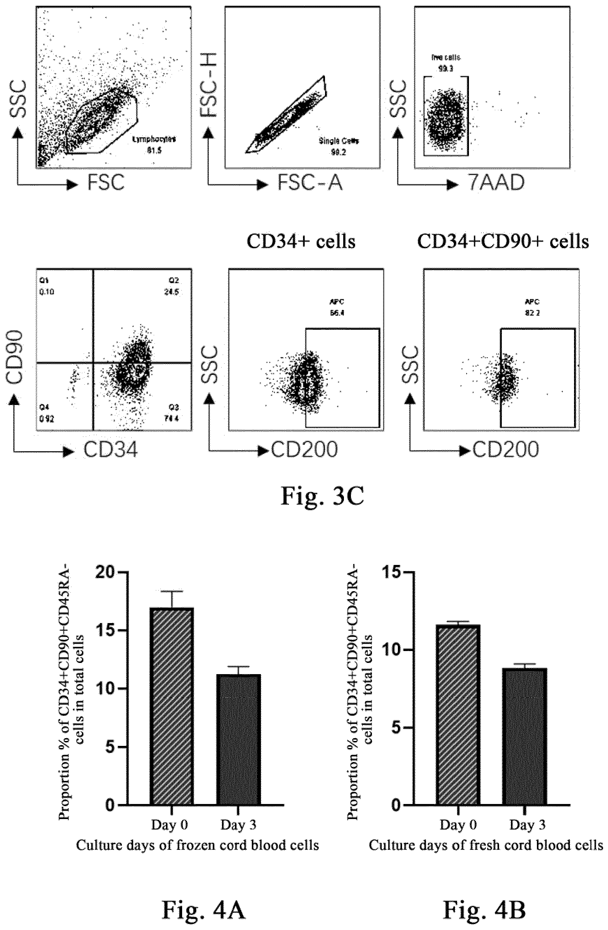

The schematic diagrams of using flow cytometry to detect the expression of CD66 (a, c, d, e), CD48, and CD200 on human HSPCs and LT-HSCs are shown in Figures 3A to 3C.

As can be seen from Fig. 2, the expression of CD48, CD200 and CD66 (a, c, d, e) in LT-HSCs (CD34+CD90+CD45RA-) was significantly higher than that in HSPCs (CD34+), and the difference was significantly higher than that of other proteins tested, indicating that CD48, CD200, CD66a, CD66c, CD66d and CD66e are expected to become new surface marker proteins of LT-HSCs.

From Figs. 3A to 3C, it can be seen that the expression level of CD48, CD200 and CD66 (a, c, d, e) in LT-HSCs (CD34+CD90+) was significantly higher than that in HSPCs (CD34+), indicating that CD48, CD200, CD66a, CD66c, CD66d and CD66e are expected to become new surface marker proteins of LT-HSCs.

### Example 4: Verification of surface protein expression

Further screening and identification of antibodies against surface proteins of human HSCs were performed respectively on frozen or freshly isolated cord blood CD34+HSPCs and their corresponding cells cultured for three days. Cells were stained with fluorescent antibodies using the following antibodies for the candidate marker proteins (Table 2) in conjunction with the antibodies used to identify cell phenotypes (Table 3), 100000 cells/100 µl per condition. After staining for 15 minutes at 4°C in dark, 2 µl of 7AAD (Biolegend, Code: 420404) was added to each well, followed by staining at 4°C in dark for 5 minutes, so as to identify the cell viability. After staining, each tube was washed by adding 1 ml of the flow cytometry staining solution, followed by centrifugation at 400 g for 5 min, and the cells were resuspended with 100 µl of the flow cytometry staining solution. The sample was loaded on a flow cytometer (Beckman Coulter CytoFLEX) to detect fluorescent expression. The expression of each surface protein was represented by ratio of positive cells and an average fluorescence intensity (MFI).

**Table 2: Specific information of antibodies of candidate marker proteins**

| Protein name | Fluorescent label | Brand | Item No. | Use concentration (per 100 µl) |
|---|---|---|---|---|
| CD48 | PE | Yiqiao Shenzhou | 10797-R248-P | 5 µl |
| CD66a | PE | Yiqiao Shenzhou | 10822-MM02-P | 5 µl |
| CD66c | PE | Yiqiao Shenzhou | 10823-MM12-P | 5 µl |
| CD66d | PE | Yiqiao Shenzhou | 11933-MM06-P | 10 µl |
| CD66e | PE | Yiqiao Shenzhou | 11077-MM02-P | 2 µl |
| CD200 | PE | Yiqiao Shenzhou | 10886-MM17-P | 5 µl |

**Table 3: Specific information of antibodies used to identify cell phenotypes**

| Protein name | Fluorescent label | Brand | Item No. | Use concentration (per 100 µl) |
|---|---|---|---|---|
| CD34 | BV510 | Biolegend | 343528 | 5 µl |
| CD90 | APC | Biolegend | 328114 | 5 µl |
| CD45RA | FITC | Biolegend | 304106 | 5 µl |

Fig. 4A-4B are schematic diagrams of the differences in LT-HSCs contained in cultured and uncultured human hematopoietic stem/progenitor cells.

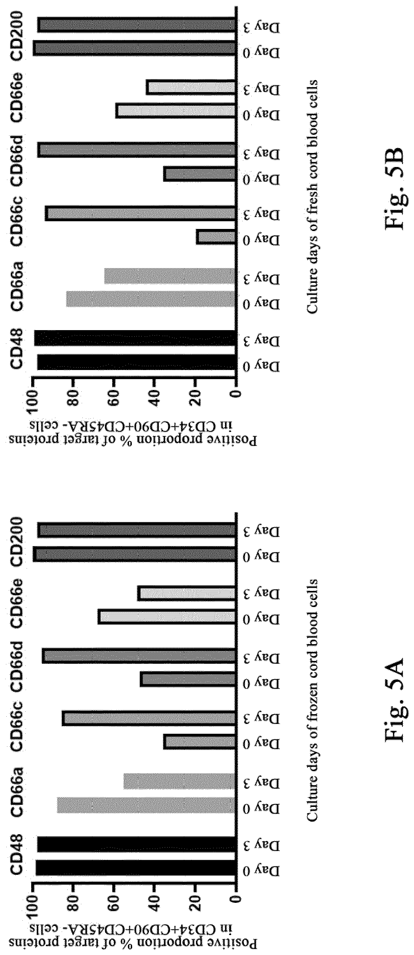

Figs. 5A-5B are schematic diagrams of re-screening for surface marker proteins on HSPCs from different sources.

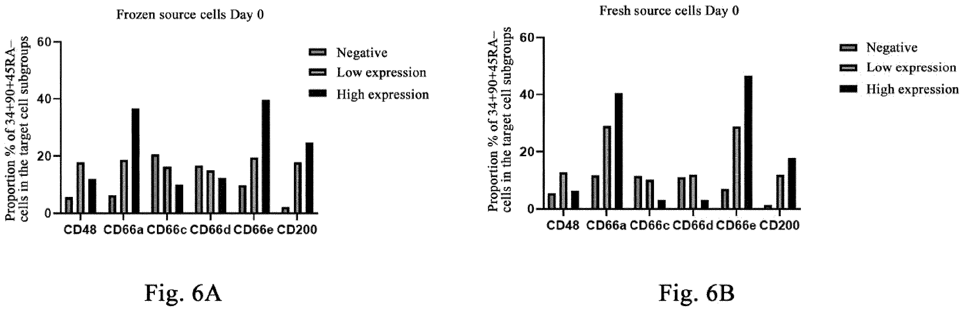

Figs. 6A-6B are schematic diagrams of distinguishing different sub-populations of HSPCs and analyzing the proportions of LT-HSCs thereof using target proteins.

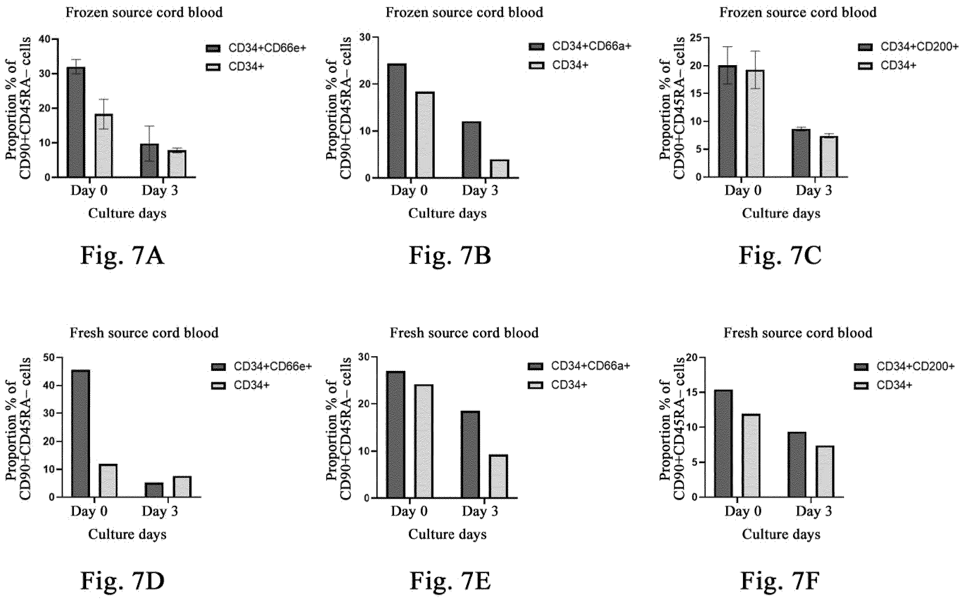

Figs. 7A-7F are schematic diagrams comparing the proportions of LT-HSCs contained in CD200, CD66e, CD66a sub-populations over culture time.

As can be seen from Figs. 4A and 4B, although HSPCs maintained growth in the *in vitro* culture system, it did not maintain good stemness, and thus the proportion of LT-HSCs decreased after 3 days of culture.

It can be seen from Figs. 5A and 5B that the expression changes of the target proteins in cord blood cells from frozen and fresh sources before and after three-day *in vitro* cultivation, and it is found that the proportions of CD66e and CD66a in LT-HSCs decrease with *in vitro* cultivation, which is similar to the decreasing trend of LT-HSCs; there was also a very slight decrease in CD200.

As can be seen from Figs. 6A and 6B, the sub-populations with higher expression levels of CD66a, CD66e and CD200 in cord blood cells from frozen and fresh sources were enriched with more LT-HSCs (CD34+CD90+CD45RA-).

It can be seen from Figs. 7A-7F that CD34+ HSPCs without *in vitro* culture contain 10-25% LT-HSCs, and the proportion of LT-HSCs is similar or slightly increased in CD34+CD200+ and CD34+CD66a+ cells; the proportion of LT-HSCs in CD34+CD66e+ cells was significantly higher, and could even exceed 40% in fresh cord blood cells.

### Example 5: Verification of HSC-related gene expression

First, specific cell sub-populations were obtained by flow cytometry sorting. Cord blood CD34+ cells were subjected to fluorescent antibody staining (Table 4). After staining for 15 min at 4°C in dark, 2 µl of 7AAD (Biolegend, item No: 420404) was added to each well to further stain at 4°C in dark for 5 minutes to identify the cell viability. After staining, each tube was washed by adding 1 ml of the flow cytometry staining solution. After centrifugation at 400 g for 5 min, the cells were resuspended with 100 ul of the flow cytometry staining solution and two groups of CD34+CD90+CD45RA-CD66e- and CD34+CD90+CD45RA-CD66e hi (CD66e hi refers to CD66e high expressing cells) cells were sorted on a flow cytometry (BD, aria III).

The sorted cells were then subjected to cell phenotypic purity determination on a flow cytometry (Beckman Coulter CytoFLEX).

The obtained cells were subjected to RNA extraction, and then reversely transcribed into cDNA (Full Gold One Step cDNA Reverse Transcription Kit, AT311-03). qRT-PCR reaction (Roche 6402712001) was performed according to the reaction systems (Table 5) and the reaction procedures (Table 6). The results were expressed using GAPDH as the reference gene and 2^-ΔΔCT to represent the expression of each gene.

**Table 4: Specific information of antibodies used for flow cytometry sorting**

| Protein name | Fluorescent label | Brand | Item No. | Use concentration (per 100 µl) |
|---|---|---|---|---|
| CD34 | BV510 | Biolegend | 343528 | 5 µl |
| CD90 | APC | Biolegend | 328114 | 5 µl |
| CD45RA | FITC | Biolegend | 304106 | 5 µl |
| CD66e | PE | Yiqiao Shenzhou | 11077-MM02-P | 2 µl |

**Table 5: qRT-PCR reaction system**

| Components | Use |
|---|---|
| F primer | 1 µl |
| R primer | 1 µl |
| MIX | 10 µl |
| cDNA | 2 µl |
| H₂O | 6 µl |

**Table 6: qRT-PCR reaction procedures**

| Temperature | Time | Number of Cycles |
|---|---|---|
| 95°C | 10 min | 1 Cycle |
| 95°C | 30 sec | 40 Cycle |
| 63°C | 30 sec | |
| 72°C | 15 sec | |
| 95°C | 10 sec | 1 Cycle |
| 65°C | 1 min | Heating slope 2.2°C/S |
| 95°C | 1 sec | |

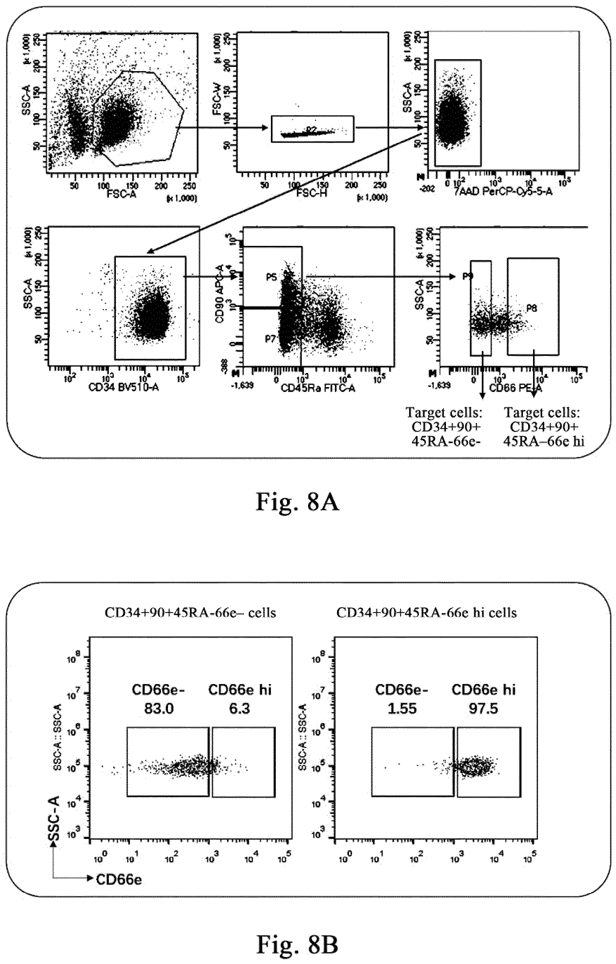

Figs. 8A and 8B are schematic diagrams of flow cytometry analysis of CD34+CD90+CD45RA-**CD66e-** or CD34+CD90+CD45RA-CD66e hi cells and their cell purity after sorting.

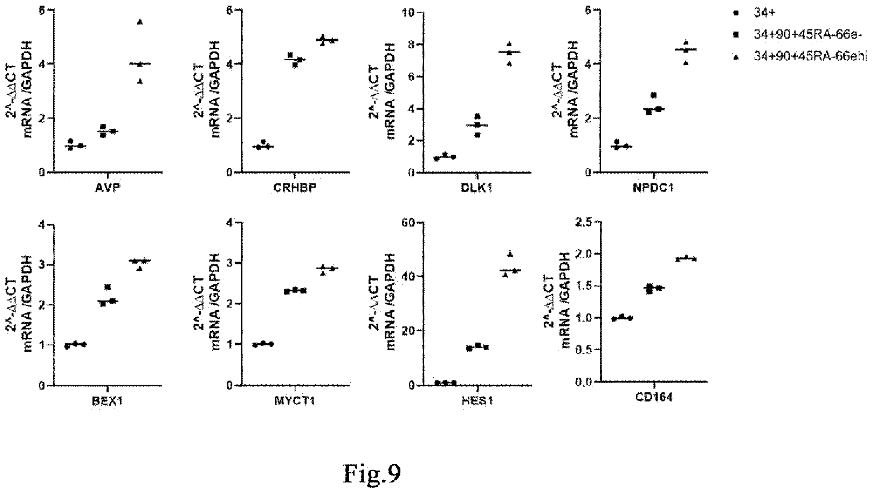

Fig. 9 is a schematic diagram of HSC-related gene expression detection in CD34+, CD34+CD90+CD45RA-CD66e-, and CD34+CD90+CD45RA-CD66e hi cells using qRT-PCR, respectively.

It can be seen from Fig. 8B that the purity of CD34+CD90+CD45RA-CD66e hi cells after flow cytometric sorting reached 97.5%.

As can be seen from Fig. 9, the expression levels of all the HSC-related genes measured were the highest in CD34+CD90+CD45RA-CD66e hi cells, indicating that CD34+CD90+CD45RA-CD66e hi cells were enriched with more HSCs.

### Example 6

Human CD34+ HSPCs were resuscitated, the cells were collected after overnight culture in a complete culture medium of hematopoietic stem cells and stained with anti-human CD34, CD90, CD45RA and CD66e fluorescent antibodies, and sorted to obtain two groups of cells by flow cytometry: CD66e+ group: CD34+CD90+CD45RA-CD66e+, and CD66e- group: CD34+CD90+CD45RA-CD66e-. Two groups of cells were transplanted into 1.0Gy irradiated NPG (NOD-scid I12rg-/-) immunodeficient mice, each mouse was transplanted with 300, 1000 or 3000 cells, respectively, 8 mice per group. The efficiency of cell transplantation was measured in peripheral blood at the 4th, 12th and 16th weeks after transplantation, and in both bone marrow and spleen of the mice at the 16th week. Transplant efficiency = hCD45⁺ proportion/(hCD45⁺ proportion + mCD45⁺ proportion) x100%, limited dilution method is to use the ELDA online tool http://bioinf.wehi.edu.au/software/elda/, to calculate the proportion of HSCs with the reconstitution ability included in each cell sub-population.

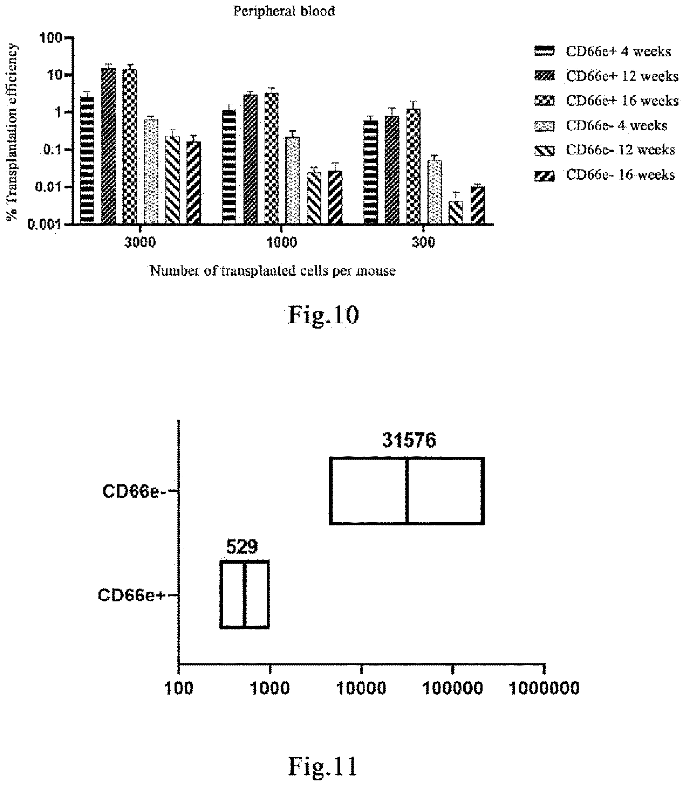

Fig. 10 is a graph showing the statistical analysis of cell transplantation efficiency in peripheral blood at 4, 12 and 16 weeks respectively after cell transplantation into mice.

Fig. 11 is a schematic diagram of HSCs with reconstruction in two groups of cells calculated by the limited dilution method.

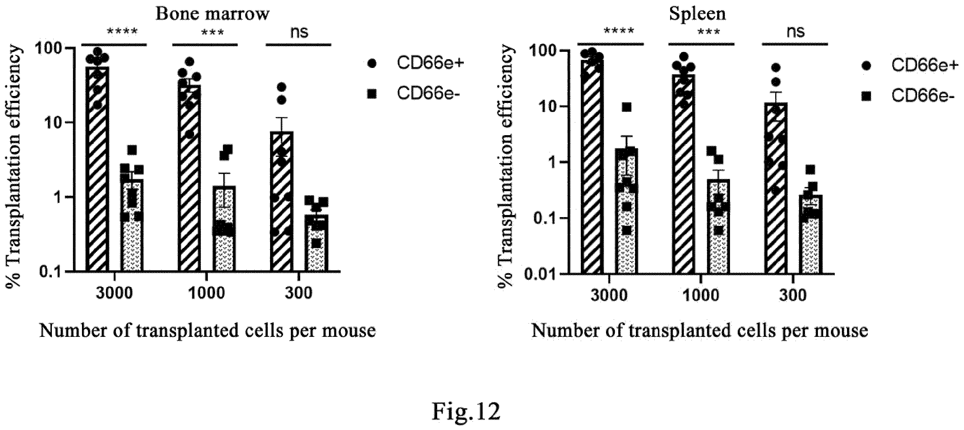

Fig. 12 is a schematic diagram of transplantation situation in bone marrow and spleen at the 16th week after transplantation.

The results in Fig. 10 show that the transplantation efficiency of CD34+CD90+CD45RA-CD66e+ cells was significantly higher than that of CD34+CD90+CD45RA-CD66e- cells, and that the transplantation efficiency increased with the number of transplanted cells.

The results in Fig. 11 show that CD34+CD90+CD45RA-CD66e+ cells contained 1/529 HSCs with reconstruction, calculated by the limited dilution method, which is 60-fold higher than that of CD34+CD90+CD45RA-CD66e- cells.

The results in Fig. 12 show that the transplantation efficiency of CD34+CD90+CD45RA-CD66e+ cells was also much higher than that of the CD34+CD90+CD45RA-CD66e- cells in the bone marrow and spleen at 16 weeks after transplantation.

### Example 7

The bone marrow of the mice 16 weeks after transplantation in Example 6 was harvested and retransplanted into NPG immunodeficient mice irradiated with 1.0 Gy. The mice were examined for transplantation situation in the bone marrow, peripheral blood and spleen at the 16th week after transplantation. Among them, transplantation efficiency = hCD45⁺ Proportion/(hCD45⁺ Proportion + mCD45⁺ Proportion) x100%.

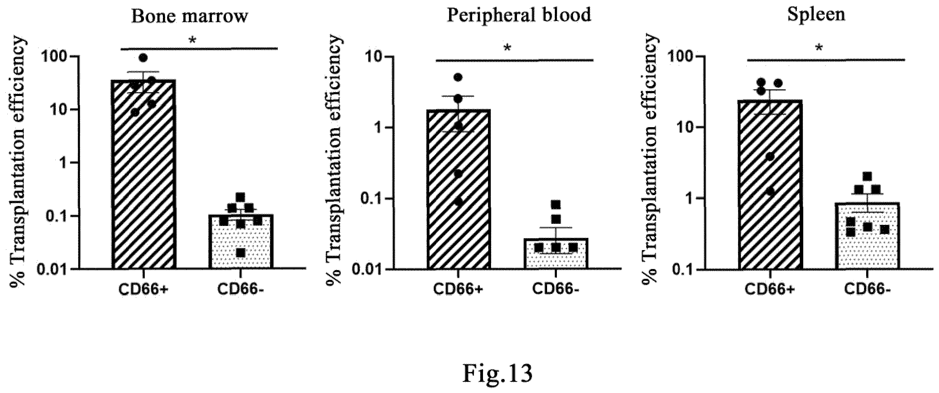

Fig. 13 is a schematic diagram of the transplantation situation at the 16th week of secondary transplantation in peripheral blood, bone marrow and spleen of mice.

The results in Fig. 13 show that CD34+CD90+CD45RA-CD66e+ cells exhibit long-term hematopoietic reconstruction function after secondary transplantation, and the transplantation efficiency was significantly higher than that of CD34+CD90+CD45RA-CD66e- cells.

In summary, it is found that all the aforementioned proteins have the potential to identify hematopoietic stem cells such as LT-HSCs, especially CD66e, CD66a and CD200. In addition, CD66e may serve as a novel surface marker protein for human LT-HSCs.

The above described are only preferred embodiments of the present application and is not intended to limit the present application in any other form. Those skilled in the art may use the technical content disclosed above to alter or modify them into equivalent embodiments of the equivalent changes. However, any simple modifications, equivalent changes, or modifications made to the above examples based on the technical essence of the present application, which do not deviate from the content of the technical solution of the present application, still fall within the scope of protection of the technical solutions of the present disclosure.

## Claims

1. Use of a protein in identifying hematopoietic stem cells, wherein the protein comprises
(1) CD34, CD90 and CD45RA; and
(2) one or two or more protein(s) selected from the group consisting of CD48, CD66a, CD66c, CD66d, CD66e and CD200.

2. The use according to claim 1, wherein the protein comprises
(1) CD34, CD90 and CD45RA; and
(2) CD66a, CD66e and/or CD200, preferably CD66e.

3. A method for identifying hematopoietic stem cells, comprising the following steps:
detecting whether the cells to be tested are CD34 positive;
detecting whether the cells to be tested are CD90 positive;
detecting whether the cells to be tested are CD45RA negative; and
detecting whether the cells to be tested are positive for one or two or more protein(s) selected from the group consisting of CD48, CD66a, CD66c, CD66d, CD66e and CD200, and
identifying the cells to be tested as hematopoietic stem cells if the cells to be tested are CD34 positive, CD90 positive, CD45RA negative and positive for one or two or more protein(s) selected from the group consisting of CD48, CD66a, CD66c, CD66d, CD66e and CD200.

4. The method according to claim 3, wherein the cells to be tested are derived from cord blood, recruited peripheral blood, or bone marrow.

5. The method according to claim 3 or 4, wherein the protein(s) is CD66a, CD66e, and/or CD200, preferably CD66e.

6. A method for screening hematopoietic stem cells, comprising:
screening hematopoietic stem cells of CD34+, CD90+, CD45RA- and positive for protein from cells to be isolated;
wherein the protein is one or two or more protein(s) selected from the group consisting of CD48, CD66a, CD66c, CD66d, CD66e and CD200.

7. The method according to claim 6, wherein the cells to be isolated are derived from cord blood, recruited peripheral blood or bone marrow.

8. The method according to claim 6 or 7, wherein the protein(s) is CD66a, CD66e, and/or CD200, preferably CD66e.

9. A cell preparation, comprising hematopoietic stem cells sorted from cells and having CD34+, CD90+, CD45RA- and being positive for one or two or more protein(s) selected from a group consisting of CD48, CD66a, CD66c, CD66d, CD66e and CD200.

10. The cell preparation according to claim 9, wherein the cells are derived from cord blood, recruited peripheral blood, or bone marrow.

11. The cell preparation according to claim 9 or 10, wherein the protein(s) is CD66a, CD66e, and/or CD200, preferably CD66e.

12. A method for preparing a cell preparation, comprising:
screening hematopoietic stem cells of CD34 +, CD90 +, CD45RA- and positive for one or two or more protein(s) from cells to be sorted;
wherein the protein(s) is one or two or more protein(s) selected from the group consisting of CD48, CD66a, CD66c, CD66d, CD66e and CD200.

13. The method according to claim 12, wherein the cells to be sorted are derived from cord blood, recruited peripheral blood, or bone marrow.

14. The method according to claim 12 or 13, wherein the protein(s) is CD66a, CD66e, and/or CD200, preferably CD66e.

15. A medicament for improving hematopoietic reconstruction, comprising the cell preparation according to any one of claims 9 to 11 or a cell preparation prepared by the method according to any one of claims 12 to 14.

16. A method for reconstructing the hematopoietic system of a subject, comprising administering to the subject the cell preparation according to any one of claims 9 to 11 or a cell preparation prepared by the method according to any one of claims 12 to 14.

17. A kit or product for detecting hematopoietic stem cells, comprising a compound for:
detecting whether the cells to be tested are CD34 positive;
detecting whether the cells to be tested are CD90 positive;
detecting whether the cells to be tested are CD45RA negative; and
detecting whether the cells to be tested are positive for one, two, three, four or more protein(s) selected from the group consisting of CD48, CD66a, CD66c, CD66d, CD66e and CD200.

18. The kit or product according to claim 17, wherein the protein is one, two or three protein(s) selected from CD66a, CD66e and CD200, preferably CD66e.

19. The kit or product according to claim 17 or 18, wherein the cells to be tested are derived from cord blood, recruited peripheral blood or bone marrow.

20. The kit or product according to any one of claims 17-19, wherein the compound is an antibody.

21. The kit or product according to claim 20, wherein the antibody is a chemically labelled antibody, such as a luminescent compound labelled antibody, such as a fluorescent labelled antibody.

22. The kit or product according to claim 20, wherein the antibody is an antibody attached to a magnetic bead.

23. A kit or product for sorting or enriching hematopoietic stem cells, comprising a compound for sorting hematopoietic stem cells from cells, wherein the hematopoietic stem cells are CD34+, CD90+, CD45RA-, and positive for one, two, three, four or more protein(s) selected from a group consisting of CD48, CD66a, CD66c, CD66d, CD66e and CD200.

24. The kit or product according to claim 23, wherein the protein is one, two or three proteins selected from a group consisting of CD66a, CD66e and CD200, preferably CD66e.

25. The kit or product according to claim 23 or 24, wherein the cells are derived from cord blood, recruited peripheral blood or bone marrow.

26. The kit or product according to any one of claims 23-25, wherein the compound is an antibody.

27. The kit or product according to claim 26, wherein the antibody is a chemically labelled antibody, such as a luminescent compound labelled antibody, such as a fluorescent labelled antibody.

28. The kit or product according to claim 26, wherein the antibody is an antibody attached to a magnetic bead.

29. A method for screening a culture medium for hematopoietic stem cells, comprising culturing hematopoietic stem cells in a culture medium to be screened, and detecting maintenance or elevation of proportion of hematopoietic stem cells **characterized by** CD34+, CD90+, CD45RA-, and positive for one, two, three, four or more protein(s) selected from the group consisting of CD48, CD66a, CD66c, CD66d, CD66e and CD200, wherein the maintenance or elevation of proportion of hematopoietic stem cells **characterized by** CD34+, CD90+, CD45RA-, and positive for one, two, three, four or more protein(s) selected from the group consisting of CD48, CD66a, CD66c, CD66d, CD66e and CD200 after cultivation indicates that the culture medium to be screened is a suitable culture medium for hematopoietic stem cells.

30. A culture medium for hematopoietic stem cells screened by the method according to claim 29.

31. A method for treating a disease in a subject, comprising administering to the subject an effective amount of the cell preparation according to any one of claims 9 to 11 or a cell preparation prepared by the method according to any one of claims 12 to 14.
